# EUROPÄISCHE PATENTSCHRIFT

(11) **EP 1 200 831 B1**
(45) Veröffentlichungstag und Bekanntmachung des Hinweises auf die Patenterteilung: **23.11.2005**
(21) Anmeldenummer: 00949423.8
(22) Anmeldetag: 29.07.2000
(51) Int. Cl.: G01N 33/68

(54) **MODULATORISCHE BINDUNGSSTELLE AN KALIUMKANÄLEN ZUM SCREENING**
MODULATING BINDING SITE ON POTASSIUM CHANNELS USED FOR SCREENING
SITE DE FIXATION DE MODULATION SELECTIVE DE CANAUX DE POTASSIUM A DES FINS D'ANALYSE

(30) Priorität: 03.08.1999 US 368314
(43) Veröffentlichungstag der Anmeldung: 02.05.2002
(73) Patentinhaber: elbion AG, 01445 Radebeul (DE)
(72) Erfinder: RUNDFELDT, Chris, D-01640 Coswig (DE); NETZER, Rainer, D-22549 Hamburg (DE)
(74) Vertreter: Böhm, Brigitte
(86) Internationale Anmeldenummer: PCT/EP2000/007348
(87) Internationale Veröffentlichungsnummer: WO 2001/009612

(56) Entgegenhaltungen:
- WO-A-99/07832
- WO-A-99/21875
- WO-A-99/31232
- US-A- 5 328 830
- RUNDFELDT CHRIS ET AL: "The novel anticonvulsant retigabine activates M-currents in Chinese hamster ovary-cells tranfected with human KCNQ2/3 subunits." NEUROSCIENCE LETTERS, Bd. 282, Nr. 1-2, 17. März 2000 (2000-03-17), Seiten 73-76, XP000972246 ISSN: 0304-3940
- SCHROEDER BJORN C ET AL: "Moderate loss of function of cyclic-AMP-modulated KCNQ2/KCNQ3K+ channels causes epilepsy." NATURE (LONDON), Bd. 396, Nr. 6712, 17. Dezember 1998 (1998-12-17), Seiten 687-690, XP002154361 ISSN: 0028-0836 in der Anmeldung erwähnt
- WANG HONG-SHENG ET AL: "KCNQ2 and KCNQ3 potassium channel subunits: Molecular correlates of the M-channel." SCIENCE (WASHINGTON D C), Bd. 282, Nr. 5395, 4. Dezember 1998 (1998-12-04), Seiten 1890-1893, XP002900985 ISSN: 0036-8075 in der Anmeldung erwähnt
- RUNDFELDT CHRIS: "Characterization of the K+ channel opening effect of the anticonvulsant retigabine in PC12 cells." EPILEPSY RESEARCH, Bd. 35, Nr. 2, Juni 1999 (1999-06), Seiten 99-107, XP000972218 ISSN: 0920-1211 in der Anmeldung erwähnt
- WICKENDEN ALAN D ET AL: "Retigabine, a novel anti-convulsant, enhances activation of KCNQ2/Q3 potassium channels." MOLECULAR PHARMACOLOGY, Bd. 58, Nr. 3, September 2000 (2000-09), Seiten 591-600, XP000972243 ISSN: 0026-895X
- MAIN MARTIN J ET AL: "Modulation of KCNQ2/3 potassium channels by the novel anticonvulsant retigabine." MOLECULAR PHARMACOLOGY, Bd. 58, Nr. 2, August 2000 (2000-08), Seiten 253-262, XP000972245 ISSN: 0026-895X

## Beschreibung

Die Erfindung betrifft eine neue selektive modulatorische Bindungsstelle an Kaliumkanälen zum Screening und Auffinden von neuen Wirkstoffen zur Behandlung von Erkrankungen, die auf eine Übererregung oder mangelnde Erregbarkeit von neuronalen Zellen zurückzuführen sind.

Kaliumkanäle haben ausgesprochen vielfältige Funktionen in elektrisch erregbaren und nicht erregbaren Zellen. Zu diesen Funktionen gehört unter anderem die Kontrolle des Membranpotentials, die Regulation der insulinsekretion aus pankreatischen β-Zellen, die Steuerung der Freisetzung von Zytokinen aus T-Lymphozyten, die Regulation des Salz- und Wasserhaushalts in Nierenzellen und die Steuerung der elektrischen Erregbarkeit und synaptischen Plastizität in Neuronen.
Es ist deshalb nicht überraschend, daß die Aktivität von Kaliumkanälen einer Vielzahl von Steuerungsmechanismen unterliegt, wozu das Redoxpotential einer Zelle, die sekundären Botenstoffe Kalzium und cyclo-Adenosinmonophosphat (c-AMP), Proteinkinasen und - phosphatasen, und das Membranpotential gehören.
Zudem weisen die Strukturen der bisher charakterisierten Kaliumkanalproteine vielfältige Varianten von verschiedenen Grundmotiven auf und sind damit sehr heterogen. Aufgrund dieser vielfältigen Funktionen ist es auch nicht verwunderlich, daß verschiedene Kaliumkanäle ubiquitär im Pflanzen- und Tierreich vorkommen (1).

Durch diese vielfältigen Strukturen in Verbindung mit der sehr großen Bandbreite der Modulationswege werden Kaliumkanäle selektiv ihren heterogenen Aufgaben gerecht. Substanzen, die Kaliumkanäle selektiv modulieren, sind interessante Medikamente für eine Vielzahl von unterschiedlichen Erkrankungen. In der Literatur werden Kaliumkanäle als Targets für die Behandlung des Schlaganfalls, der Epilepsie, der Alzheimer'schen Erkrankung, bei psychiatrischen Erkrankungen, bei Störungen des Schlafs, bei Rhythmusstörungen des Herzens, bei Diabetes Typ II, bei osmotischen Dysfunktionen wie bei Glaukom, bei Tumorzellwachstum, aber auch bei Entzündungsprozessen und bei Lemleistungsstörungen, bei Bluthochdruck, Blasenschwäche und Asthma diskutiert.
Bisher konnte die Diabetesbehandlung und die Behandlung von Rhythmusstörungen des Herzens und Bluthochdruck mit selektiven Medikamenten mit Erfolg implementiert werden (1).

1998 berichteten Schröder et al. zum erstenmal, daß eine mutationsbedingte geringfügige Beeinflussung nur eines Mitglieds der großen Familie der Kaliumkanäle das fragile Gleichgewicht zwischen Erregung und Hemmung bei erregbaren Zellen substantiell stören kann. Bei diesem bestimmten Kanal handelt es sich um ein Heterooligomer aus den Untereinheiten mit der Bezeichnung KCNQ2 und KCNQ3. Durch Mutation von einer der beiden Untereinheiten wird die Funktion dieses Kanals um ca. 25% reduziert. Dies führt bei den betroffenen Patienten dazu, daß sie bereits im Säuglingsalter an epileptischen Anfällen leiden (2).
Dieser Kanal ist der erste Kaliumkanal, der eindeutig einer Erkrankung des Menschen zugeordnet werden konnte.
Schröder postulierte, daß eine positive Modulation dieses Kanals eine starke antikonvulsive Wirkung hervorrufen sollte. Er schlußfolgerte, daß durch Steigerung des Spiegels des intrazellulären Botenstoffes cAMP die Aktivität des Kanals positiv beeinflußt wird; er konnte in seinen in vitro Systemen zeigen, daß bei gesteigerten cAMP-Spiegel tatsächlich die Aktivität des Kanals zunahm.

Etwa zur gleichen Zeit zeigten Wang et al. (3), daß der oben erwähnte beim Menschen nachgewiesene heterooligomere Kanal bestehend aus KCNQ2 und KCNQ3 das molekulare Korrelat des funktionell seit langem beschriebenen M-Kanals ist.

Der M-Kanal wird selektiv nur in neuronalen Zellen ausgebildet (2, 3) und ist dort über intrazelluläre Signalproteine (G-Proteine) selektiv im Zentralnervensystem an muskarinerge Subtypen des Acetylcholinrezeptors gekoppelt. In peripherem Gewebe wird der Kanal nicht exprimiert. Während Muskarinagonisten die Aktivität des Kanals senken, kann diese durch Muskarinantagonisten gesteigert werden.
Der Muskarinagonist Pilocarpin löst beim Tier schwere Krämpfe aus. Der daraus resultierende Untergang der Zellen, in denen der Muskarinrezeptor (und damit auch den M-Kanal) exprimiert ist führt dazu, daß die Tiere nach dieser Behandlung spontane Epilepsien entwickeln.
Durch einen anderen Muskarinagonisten, Oxotremorin, läßt sich beim Tier in subletalen Dosen ein essentieller Tremor auslösen, der dem Tremor des Parkinsonpatienten ähnelt. Muskarinantagonistische Substanzen werden zur Behandlung dieses Tremors klinisch eingesetzt.
Aus diesen exemplarischen Darstellungen wird deutlich, daß eine indirekte (über Muskarinrezeptoren ausgelöste oder durch Steigerung der cAMP-Spiegels hervorgerufene) Modulation des M-Kanals eine hoch interessante Möglichkeit darstellt, in verschiedene Erkrankungen einzugreifen.

Im Einzelnen soll dies ausführlicher für mehrere Erkrankungen dargestellt werden.

### Epilepsie

Die Erkrankung Epilepsie ist durch das wiederholte Auftreten von Krampfanfällen charakterisiert. Sie tritt in der Befölkerung mit einer Prävalenz von 0.5-1 % auf. Die epileptischen Krampfanfälle resultieren aus einer abnormen Synchronisation und massiven Entladung einer großen Anzahl von Nervenzellen in einem Nervenzellverband im Gehirn. Je nach Beteiligung verschiedener Gebiete des Gehirns resultiert dies in einer anfallsartigen vorübergehenden Störung der Bewegung (motorische Krämpfe), der Empfindung, des Verhaltens oder der Wahrnehmungsfähigkeit (4).

Der Krampfanfall ist jedoch nur ein Symptom, das prinzipiell bei jedem Individuum ausgelöst werden kann, wenn der Stimulus zur Aktivierung und Synchronisation ausreichend stark ist. Die Erkrankung Epilepsie ist daher durch die erhöhte Empfindlichkeit auf äußere oder innere Reize zur Synchronisation und Aktivierung charakterisiert. Die Erregbarkeit und damit die Empfindlichkeit von Nervenzellen ist bei epileptischen Patienten größer als bei nicht epileptischen Patienten.

Kürzlich konnte, wie bereits erwähnt, gezeigt werden, daß Kaliumkanäle, die aus den Untereinheiten KCNQ2 und KCNQ3 zusammengesetzt sind, entscheidend die Erregbarkeit von Nervenzellen steuern (2).
Bereits eine Reduktion der Funktion dieser Kanäle um etwa 25% führt bei Säuglingen, die nicht über ausreichende kompensatorische Mechanismen verfügen, zu Epilepsie. Eine solche schwache Reduktion der Funktion dieses Kaliumkanals wurde für eine genetisch aufgeklärte Form der Epilepsie, die BFNC (benign familial neonatal convulsions, benigne familiäre Neugeborenenkrämpfe), nachgewiesen (2).
Dieser Kaliumkanal ist nur im Gehirn und in Nervenzellen nachweisbar, nicht aber in anderen Geweben (2, 3).
Bei anderen Formen der Epilepsie konnte die genetische Ursache noch nicht aufgeklärt werden, jedoch geht die Erkrankung immer mit einer erhöhten Erregbarkeit von Nervenzellverbänden einher. Die bisher übliche Therapie setzt symptomatisch an. Eingeführte Antiepileptika wie Carbamazepin, Phenytoin und Lamotrigin wirken als nutzungsabhängige Blocker von Natriumkanälen. Diese Kanäle sind notwendig um zelluläre Erregungen entlang der Nervenfasern weiterzuleiten.
Natriumkanalblocker reduzieren die Weiterleitung von Erregungen und wirken auf diese Art antikonvulsiv. Die zugrundeliegende Übererregung wird nicht reduziert. Andere Antiepileptika wie Phenobarbital, Clonazepam, Vigabatrin, Topiramat oder Valproat verstärken die hemmende Nervenübertragung oder reduzieren die erregende Nervenübertragung, d.h. verringern die Wahrscheinlichkeit, daß eine Erregung auf andere Nervenzellen übertragen wird. Auch hier wird die Ursache der Erkrankung, die zugrundeliegende Übererregbarkeit einzelner Nervenzellen, nicht beeinflusst, sondern nur die Singalerweiterung reduziert. Medikamente, die jedoch selektiv den oben erwähnten Kaliumkanal positiv modulieren, können direkt die Erregbarkeit von Nervenzellen beeinflussen. Da der Kanal nur in neuronalem Gewebe nachweisbar ist, ist eine selektive Wirkung ohne Nebenwirkungen auf andere Gewebe für solche Substanzen zu erwarten.

### Morbus Alzheimer

Wie erwähnt stellt der Kaliumkanal bestehend aus den Untereinheiten KCNQ2 und KCNQ3 das molekulare Korrelat des M-Kanals dar (3). Der M-Kanal ist über eine enge Kopplung an einen Subtyp des Acetylcholinrezeptors, den Muskarinrezeptor des zentralen Nervensystems negativ geokppelt. Muskarinrezeptoren außerhalb des Nervensystems sind nicht mit dem M-Kanal gekoppelt. Eine Aktivierung von Muskarinrezeptoren führt zu einer Reduzierung der Öffnungswahrscheinlichkeit dieses Kanals, also zu einer Reduktion der Funktion.

Dies wird bereits pharmakologisch ausgenutzt. Durch schwache Hemmung des Acetylcholin-abbauenden Enzyms Acetylcholinesterase mittels Medikamenten wie Donepezil-HCl (Aricept®) wird die Acetylcholinkonzentration im Gehirn erhöht, der Muskarinrezeptor aktiviert und dadurch der Kaliumkanal negativ moduliert (gehemmt).
Dies hat eine Erhöhung der Erregbarkeit von cholinergen Nervenzellen zur Folge. Beim Morbus Alzheimer gehen selektiv cholinerge Nervenzellen zu Grunde, was zu den bekannten Symptomen des Gedächtnisverlust (Demenz) führt. Durch Erhöhung der Erregbarkeit der verbleibenden cholinergen Nervenzellen können diese die Funktion der untergegangenen Nervenzellen ausgleichen, wodurch dem Gedächtnisverlust entgegengewirkt wird.

Jedoch haben die Enzymhemmer der Cholinesterase entscheidende Nachteile. Da cholinerge Signalübertragung im ganzen Körper eine wichtige Rolle spielt, so auch im Skelettmuskel und in anderen Geweben, und da die Substanzen die Cholinesterase überall hemmen, kommt es zu einer Vielzahl von Nebenwirkungen wie Schwindel, Dyspepsie, Bauchschmerzen, Übelkeit und/oder Erbrechen, Diarrhö, Anorexie, Myalgie. Gelegentlich treten auch Schwäche, Ataxie, Schlaflosigkeit, Gewichtsabnahme und Bradykardie auf.

Um diese Nachteile zu umgehen, befindet sich die Substanz Linopirdine in der Entwicklung, welches nicht das cholinerge System beeinflußt, sondern den M-Kanal (= KCNQ2 + KCNQ3) blockiert (3).
Jedoch hat dieses Medikament den Nachteil, daß es keine ausreichende Selektivität gegenüber dem M-Kanal aufweist.
Es werden mit ähnlicher Affinität Kanäle blockiert, die in der Funktion des Herzmuskels eine große Rolle spielen, insbesondere der Kanal KCNQ1, aber auch die Kanäle eag1, erg1, erg3, elk1, Kv1.2 und Kv4.3, die im Herzen und in anderen Geweben weit verbreitet sind (3).

Das Ziel der selektiven Hemmung von KCNQ2/3 konnte daher mit Medikamenten, die die Linopirdin-Bindungsstelle modulieren, nicht erreicht werden.
Auch der neue Ligand für die Linopridin-Bindungsstelle, XE991, hemmt mit ähnlicher Affinität die Kanäle KCNQ1 und Kv4.3, die beide für die Herzfunktion von Bedeutung sind. Eine Blockade dieser herzspezifischen Kanäle kann fatale Rhythmusstörungen auslösen, so daß eine Weiterentwicklung dieser Substanzen fraglich ist.

### Morbus Parkinson

Auch bei anderen Erkrankungen wird eine Modulation von zentralen Muskarinrezeptoren zur Behandlung angestrebt.
So werden Muskarinrezeptorantagonisten z.B. beim Morbus Parkinson zur Behandlung der Krankheitssymptome, vor allem des Tremors eingesetzt.
Wie beim Morbus Alzheimer ist jedoch auch hier die Selektivität von Muskarinrezeptorliganden für zentrale Rezeptoren nicht ausreichend und es kommt durch Interaktionen mit peripheren Muskarinrezeptoren und anderen Ionenkanälen und Rezeptoren zu unerwünschten Nebenwirkungen.
Da zentrale Muskarinrezeptoren mit dem M-Kanal gekoppelt sind, können M-Kanal Aktivatoren selektiver als Muskarinantagonisten diese Funktion ausüben. Diese Medikamente wurden bisher jedoch noch nicht beschrieben.

### Neurodegenerative Erkrankungen

Neben den oben genannten Erkrankungen spielt Beeinflussung der Erregbarkeit von Nervenzellen aber auch bei neurodegenerativen Erkrankungen eine wichtige Rolle (5).
Nervenzelldegeneration tritt immer dann auf, wenn ein Ungleichgewicht zwischen Energieverbrauch und Energiezufuhr besteht.
Bei einem Schlaganfall z.B. fehlt die Blutzufuhr, die Nervenzellen sterben. Bei toxischen Übererregungen wie beim Status Epilepticus oder der amyotrophen Lateralsklerose verbrauchen die Zellen aufgrund der Übererregung vermehrt Energie und die Versorgung mit neuer Energie ist nicht mehr ausreichend (5).
Zusätzlich wird von den Zellen der Neurotransmitter Glutamat ausgeschüttet. Die Neuronen können das Membranpotential aufgrund der erhöhten Konzentration an Glutamat und der ungenügenden Energieversorgung nicht mehr aufrechterhalten und depolarisieren. Durch die Aktivierung des KCNQ2/3 Kanals werden die Zellen hyperpolarisiert und können sich von den beschriebenen Noxen erholen.
Eine selektive Senkung der Erregbarkeit von solchen Nervenzellen kann daher den Nervenzelluntergang vorbeugen. Jedoch gab es bisher außer der Senkung der Körpertemperatur keine sichere direkte Methode zur Senkung der Erregbarkeit von Nervenzellen (5).
Durch die Aufdeckung der Bedeutung des Kanals KCNQ2/KCNQ3 für die selektive Steuerung der Erregbarkeit von Nervenzellen wurde deutlich, daß eine Aktivierung dieses Kanals Nervenzellschäden vorbeugen kann.

Aus den aufgezeigten Gründen ist eine selektive Beeinflussung des Kanals mit den Untereinheiten KCNQ2 und KCNQ3 notwendig, um bei Aktivierung eine selektive z.B. antikonvulsive, anti-Parkinson und neuroprotektive Wirkung, und bei Hemmung eine selektive Gedächtnis verstärkende Wirkung zu erreichen.
Neben den erwähnten Erkrankungen sind alle Zustände beeinflußbar, die mit einer Übererregung oder mangelnden Erregung von Nervenzellen einhergehen, die den M-Kanal tragen oder in ihren Projektionsfeldern liegen.

Dieses Ziel kann mit den bisher vorhandenen Medikamenten und Behandlungsstrategien nicht erreicht werden.

Erfindungsgemäß konnte nun gezeigt werden, daß das Antikonvulsivum Retigabin (Formel 1, 5, 6) hoch selektiv den aus den Untereinheiten KCNQ2 und KCNQ3 bestehenden Kanal aktiviert, d.h. positiv moduliert.

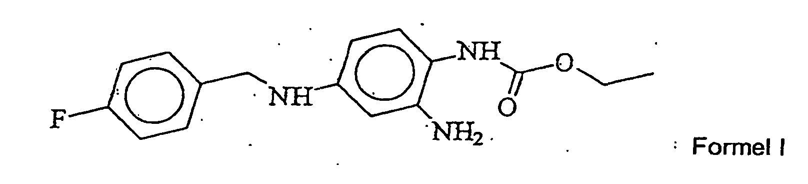

Bisher war eine direkte positive Modulation dieses Kaliumkanals nicht möglich..
Es waren keine modulatorischen Bindungsstellen bekannt, an denen Substanzen angreifen können um den Kanal positiv oder negativ zu modulieren.
Substanzen wie Linopirdine und XE991 (3), waren nur in der Lage den Kanal zu blockieren.
Diese Substanzen sind zudem nicht ausreichend selektiv, da sie wahrscheinlich im Bereich der Kanalpore angreifen, ein Bereich, der bei allen Kaliumkanälen ähnlich und für die Selektivität des Kanals für Kaliumionen verantwortlich ist.
Substanzen, die den Spiegel des Botenstoffes cAMP beeinflussen oder Substanzen, die den Muskarinrezeptor beeinflussen, üben nur eine indirekte, nicht selektive Wirkung auf den Kanal aus.

Die antikonvulsive Wirkung von Retigabin läßt sich auf die Kaliumkanalaktivierung zurückführen (7, 8).

Retigabin, ein 2-Amino-4-(4-fluorbenzylamino)-1-ethoxycarbonylaminobenzen und Verfahren zur Herstellung werden im EP 554 543 erstmals beschrieben.

Untersuchungen haben gezeigt, daß Retigabin keinen Effekt auf eine große Anzahl unterschiedlicher Kaliumkanäle aufweist (siehe Tabelle 1). Dies sind im einzelnen:
Kir1.1 (ROMK1), Kir2.1 (IRK1), Kir2.2 (IRK2), Kir2.3 (IRK3), Kir3.1 (GIRK1), Kir3.2 (GIRK2), Kir3.3 (GIRK3), Kir3.4 (GIRK4), TWIK1, Heteromultimere aus GIRK1 und GIRK2, aus GIRK2 und GIRK4, aus GIRK2 und Girk3, r-eag1, r-erg1, r-erg3 und ATP-abhängige Kaliumkanäke (Kir6.2).

Damit konnte mit Retigabin eine Bindungsstelle aufgezeigt werden, die für den Kanal, bestehend aus den Untereinheiten KCNQ2 und KCNQ3, hoch selektiv ist.
Retigabin aktivierte außerdem den homomeren Kanal, der ausschließlich aus der Untereinheit KCNQ2 besteht. In Zellen, die nur den homomeren Kanal aus der KCNQ3 Untereinheit expremieren, sind nur marginale spannungsabhängige Ströme meßbar.
In diesen Zellen kann kein Strom durch die Applikation von Retigabin ausgelöst werden (siehe Tabelle 1). Während heteromultimere Kanäle bestehend aus KCNQ2 und KCNQ3 als molekulares Korrelat des M-Kanals in Nervenzellen nachgewiesen werden konnten, sind homomultimere Kanäle die ausschließlich aus KCNQ2 oder KCNQ3 Untereinheiten bestehen zwar in Zellkultursystemen artifiziell herstellbar, wurden aber in vivo bisher nicht nachgewiesen. Es kann davon ausgegangen werden, daß der heteromultimere Kanal bestehend aus KCNQ2 und KCNQ3 die physiologisch vorkommende Form dieses Kaliumkanals ist und daß Retigabin selektiv diesen Kanal aktiviert.

Dies ist der erstmalige Nachweis, daß eine selektive positive Modulation dieses Kanals möglich ist, d.h. daß der Kanal eine modulatorische Bindungsstelle besitzt, die außerhalb der Kanalpore liegt.
Bindungsstellen innerhalb der Kanalpore führen bei Bindung des Liganden zu einer Blockade des Kanals.
Wir konnten zeigen, daß die Bindungsstelle für Retigabin auf der Kanaluntereinheit KCNQ2 liegt.
Homomultimere Kanäle, die nur aus der Untereinheit KCNQ2 bestehen, werden von Retigabin aktiviert, jedoch können in Nervenzellen nur heteromultimere Kanäle mit dem KCNQ2 und KCNQ3 nachgewiesen werden.

Die Wirkung von Retigabin auf diese heteromultimeren Kanäle ist sehr stark und konzentrationsabhängig.
Wird der Kanal durch intrazelluläre Zugabe von sättigenden Konzentrationen des stabilen cAMP-Analogons 8Br cAMP (10 mM) maximal positiv beeinflußt, wie in (2) beschrieben, dann kann noch immer durch Retigabin eine deutliche Aktivierung ausgelöst werden.
Wir konnten dadurch zeigen, daß die selektive Wirkung von Retigabin unabhängig vom cAMP-Gehalt der Zelle ist.
Es lassen sich daher durch Retigabin Wirkungen auslösen, die über die von Schröder et al in (2) beschriebenen Wirkungen hinausgehen.

**Tabelle 1:**

| Wirkung von Retigabin auf verschiedene klonierte Kaliumkanäle | | |
|---|---|---|
| Kaliumkanaluntereinheit | Expressionssystem | Wirkung von Retigabin 10 µM |
| Kir1.1 (ROMK1) | Krallenfrosch-Eizellen | Keine Wirkung |
| Kir2.1 (IRK1) | Krallenfrosch-Eizellen | Keine Wirkung |
| Kir2.2 (IRK2) | Krallenfrosch-Eizellen | Keine Wirkung |
| Kir2.3 (IRK3) | Krallenfrosch-Eizellen | Keine Wirkung |
| Kir3.1 (GIRK1) | Krallenfrosch-Eizellen | Keine Wirkung |
| Kir3.2 (GIRK2) | Krallenfrosch-Eizellen | Keine Wirkung |
| Kir3.3 (GIRK3) | Krallenfrosch-Eizellen | Keine Wirkung |
| Kir3.4 (GIRK4) | Krallenfrosch-Eizellen | Keine Wirkung |
| TWIK1 | Krallenfrosch-Eizellen | Keine Wirkung |
| GIRK1 & GIRK2 | Krallenfrosch-Eizellen | Keine Wirkung |
| GIRK2 & GIRK4 | Krallenfrosch-Eizellen | Keine Wirkung |
| GIRK2 & Girk3 | Krallenfrosch-Eizellen | Keine Wirkung |
| r-eag1 | CHO-Zellen | Keine Wirkung |
| r-erg1 | CHO-Zellen | Keine Wirkung |
| r-erg3 | CHO-Zellen | Keine Wirkung |
| KCNQ2 | CHO-Zellen | Schwache Aktivierung durch Aufgabe von 10 µM Retigabin (165 ± 65 pA) |
| KCNQ3 | CHO-Zellen | Keine Wirkung |
| KCNQ2 & KCNQ3 | CHO-Zellen | Sehr starke Aktivierung durch Aufgabe von 10 µM Retigabin (1527 ± 177 pA) |
| KCNQ2 & KCNQ3, in Gegenwart von stabilen cAMP-Analoga | CHO-Zellen | Starke Aktivierung durch Aufgabe von 10 µM Retigabin (1100-1400 pA) |

### Methodenbeschreibung zur Tabelle 1:

Für die Versuche an Krallenfrosch-Eizellen wurden der Erfahrungswert für erfolgreiche Transfektionen 500 pg bis 37 ng der literaturbekannten als Klon vorliegenden mittels Polymerasekettenreaktion vermehrten Erbinformation (cRNA) für den zu untersuchenden Kanal durch Druckinjektion in die Eizellen appliziert. Die Oozyten wurden nach dieser Behandlung in Nährmedium gelegt und am Leben erhalten. Nach einer Woche wurden die Eizellen einzeln in eine Ableitkammer verbracht und unter Verwendung der 2-Elektroden-Spannungsklemme elektrophysiologisch untersucht. Dabei wurden die Eizellen ausgehend von einem Haltepotential von -80 mV in 10 mV Schritten auf verschiedene Membranpotentiale zwischen -130 und +30 mV für jeweils 2 sec hyper- und depolarisiert und der resultierende Membranstrom gemessen. Diese Versuche wurden in Gegenwart von Kontrollösung sowie von 1, 10 und 100 µM Retigabin durchgeführt und die erhaltenen Strom-Spannungskurven verglichen. In keiner der untersuchten Präparationen konnte durch Retigabin die Aktivität des transfizierten Kaliumkanals beeinflußt werden.
Bei den in CHO-Zellen durchgeführten Versuchen wurde die Erbsubstanz für den zu untersuchenden Kanal auch mittels Liposomen in die Zelle eingebracht, die Ableitung mittels der Patch clamp Technik wurde 48-72 Stunden nach der Transfektion durchgeführt. Wieder wurden die Zellen auf -80 mV gehalten und in 10 mV Schritten von-100 bis +50 mV in Gegenwart von Kontrollösung oder Retigabin hyper- und depolarisiert. Zusätzlich wurde entsprechend einer bereits beschriebenen Methode (Rundfeldt, 1999b) auf Zellen, die leicht depolarisiert waren (-60 oder -50 mV) Retigabin in der Konzentration 10 µM aufgegeben und der dadurch ausgelöste Strom quantifiziert. Die letztere Methode wurde angewandt, um die in der Tabelle dargestellten durch Retigabin ausgelösten Ströme zu quantifizieren. Bei Zellen, in denen durch Aufgabe von Retigabin einen Strom aktiviert werden konnte, d.h. in Zellen, die die Untereinheit KCNQ2 oder KCNQ2 zusammen mit KCNQ3 enthielten, war die durch schrittweise Hyper/Depolarisation gewonnene Strom-Spannungskurve in ihrer Aktivierung zu negativeren Potentialen verschoben.
Daraus folgt eine frühere Öffnung des Kanals und damit verbunden eine Hyperpolarisation der Neuronen. Zur Überprüfung, ob die durch Retigabin ausgelöste Aktivierung des KCNQ2/3-Kanals (M-Kanals) noch möglich ist, wenn der Kanal wie von Schröder et al. 1988 beschrieben durch c-AMP maximal aktiviert ist, wurden in Versuchen eine sättigende Konzentration des nichthydrolysierbaren c-AMP-Analogons, 8-Brom-cAMP, in die Pipettenlösung gegeben. Auch unter diesen gesättigten Verhältnissen konnte Retigabin den Kanal stark aktivieren.

Durch Verwendung der erfindungsgemäßen modulatorischen Bindungstelle können nun neue Substanzen gesucht werden, die den oben genannten Kanal entweder positiv (aktivieren) oder auch negativ modulieren.

Voraussetzung für die Auslösung einer Wirkung ist die Bindung eines möglichen Wirkstoffes an eine körpereigene Substanz, die man als Rezeptor bezeichnet.
Durch die Bindung muß in dieser Bindungsstelle eine Veränderung der Konformation hervorgerufen werden. Die Konformationsänderung führt zu einer Funktionsänderung des betroffenen Rezeptors. Dieser Vorgang vermittelt die eigentliche Wirkung des zu prüfenden Wirkstoffes an dem Rezeptor.
Basierend auf diesen Erkenntnissen können nun verschiedene Screeningsysteme aufgebaut werden, mit denen neue noch selektivere oder stärker wirkende Liganden zur Aktivierung oder Hemmung des M-Kanals gefunden werden können.

Die einfachste Implementierung eines Screeningsystems ist ein Bindungsassay.
Hierzu werden ein spezifischer über lsotope markierter ("heißer") Ligand für die zu untersuchende Bindungsstelle, derselbe Ligand in nicht markierter ("kalter") Form sowie zu untersuchende Testsubstanzen und eine Proteinpräparation, die den Rezeptor enthält benötigt.
Zur Durchführung von Bindungsassays sind Apparaturen und Arbeitsvorschriften kommerziell erhältlich bzw. wurden vielfach beschrieben. Zunächst wird die Proteinfraktion zur Absättigung von unspezifischen Bindungsstellen mit dem kalten spezifischen Liganden in hoher Konzentration vermischt. Nach Abfiltrieren und Spülen der Proteinfraktion wird dann der heiße spezifische Ligand in definierter Menge und die zu testende Substanz in verschiedenen Konzentrationen auf die Proteinfraktion gegeben. Nach einer definierten Einwirkungszeit zur Equillibrierung beider Substanzen an der Bindungsstelle wird der Überstand schnell abfiltriert und wiederum durch Spülen nicht am Rezeptor gebundene Anteile entfernt. Durch Messen der verbleibenden Radioaktivität oder einer anderweitigen Markierung des spezifischen Liganden in der Proteinfraktion läßt sich feststellen, ob und wieviel des spezifischen Liganden durch die zu testende Substanz von der Bindungsstelle verdrängt wurde.
Wenn mit ansteigender Konzentration der Testsubstanz die Menge des spezifischen Liganden in der Proteinfraktion abnimmt, dann ist davon auszugehen, daß die Testsubstanz den spezifischen Liganden konzentrationsabhängig von seiner Bindungsstelle kompetitiv verdrängt und somit selbst ein Ligand für diese Bindungsstelle ist.
Als heißer und kalter Ligand dient markiertes bzw. unmarkiertes Retigabin oder ein durch Retigabin charakterisierter Ligand mit noch besseren physikochemischen Eigenschaften.
Bindungsassays werden in ihrer Spezifität fast ausschließlich durch den verwendeten Liganden charakterisiert; ohne spezifischen Liganden sind Bindungsassays nicht durchführbar.
Die Proteinpräparation kann verschiedene Quellen haben, muß aber immer den zu untersuchenden Rezeptor, hier den M-Kanal oder die Bindungsstelle tragende Teile des M-Kanals wie die Untereinheit KCNQ2 oder Teile davon enthalten.

Entscheidend ist, daß eine kompetitive Bindung des spezifischen Liganden an dem Protein möglich ist.
Das Protein kann
- aus nativem neuronalem Gewebe von Menschen (post mortem oder aus operativ entferntem Gewebe) oder von Tieren
- aus M-Kanal enthaltenden Zellkulturen wie der differenzierten Zellinie PC12 oder NG108-15 oder aus humanen Zellinien wie der Linie hNT
- aus Zellkulturen, die durch transiente oder stabile Transfektion (= Einbringung von genetischer Information in die Zelle mittels literaturbekannter Methoden) mit der genetischen Information zur Herstellung der Kanaluntereinheit KCNQ2 alleine oder in Kombination mit der Untereinheit KCNQ3 oder Teilen der Untereinheit KCNQ2 versehen wurden und die diese Information in funktionelles Protein übersetzen
- aus anderen Protein produzierenden Systemen wie Hefezellkulturen, Bakterienkulturen, Pflanzen oder Virenkulturen, sofern in die Hefen, Bakterien, Pflanzen oder Viren die genetische Information zur Herstellung des Rezeptors eingepflanzt wurde
gewonnen werden.

Bindungsassays haben den Vorteil, daß durch Einsatz des spezifischen Liganden nur Substanzen gefunden werden, die an dieselbe Bindungsstelle binden wie der spezifische Ligand. Die Anforderungen an die Reinheit der Proteinfraktion sind daher nicht hoch und es kann mit nativem Material wie Hirngewebe gearbeitet werden.
Bindungsassays sind jedoch zugleich in ihrer Aussage begrenzt.
So kann nur festgestellt werden, ob eine zu untersuchende Substanz an die fragliche Bindungsstelle (= Rezeptor) bindet oder nicht.

Es wird dagegen nicht untersucht, ob die Substanz durch die Bindung am Rezeptor auch eine Wirkung auslöst.

Theoretisch sind immer Substanzen denkbar, die binden und keine Wirkung auslösen (= neutrale Liganden), Substanzen die über den Rezeptor das Substrat (hier den M-Kanal) aktivieren (=Agonisten) oder Substanzen, die über den Rezeptor das Substrat hemmen (=Antagonisten).

Wie oben erwähnt sind M-Kanalagonisten unter anderem für die Epilepsie von Interesse, wohingegen M-Kanalantagonisten für z.B. den Morbus Alzheimer von Bedeutung sind.
Es ist daher notwendig, funktionelle Tests zur Charakterisierung der Wirkung der Testsubstanz zu verwenden.

Die sicherste Methode zur Charakterisierung von Rezeptorliganden besteht in der elektrophysiologischen Untersuchungen an Zellinien oder Zellkulturen wie für Retigabin beschrieben (8). Diese Untersuchungen sind sehr aufwendig, da jede einzelne Zelle unter Sichtkontrolle mittels Glaselektroden abgeleitet werden muß.
Testsübstanz und Referenz (Retigabin) werden durch spezielle Applikationssysteme auf die Zellen aufgegeben und die Beeinflussung der Zellfunktion in Abhängigkeit von der applizierten Konzentration der Testsubstanz bewertet.
In der letzten Zeit wurden jedoch vermehrt funktionelle Screeningtests mit hohem Durchsatz an Substanzen (bis mehrere Tausend Substanzen pro Stunde, High Throughput Screening, HTS) beschrieben.
Da hier im Gegensatz zum Bindungsassay die Zellfunktion im Vergleich zur Wirkung des spezifischen Liganden (hier Retigabin oder ein durch Retigabin charakterisierter Ligand mit noch besseren physikochemischen Eigenschaften) bewertet wird, ist es notwendig, daß die zu untersuchende Bindungsstelle in genetisch definierter Form und zugleich als funktionelle Einheit in dem System vorliegt. Bei dem System kann es sich um Zellkulturen oder um künstlich hergestellte zellähnliche Konstrukte (Lipiddoppelmembranen) mit funktionell eingelagertem Rezeptor/Kanal handeln. Die Zellkulturen müssen den zu untersuchenden Rezeptor (hier den M-Kanal) sicher und reproduzierbar exprimieren und funktionell in die Membran einbauen.
Weiterhin muß ein Reportersystem in die Zellen bzw. zellähnlichen Konstrukte eingebracht werden, welches eine Aktivierung oder eine Inaktivierung des Kanals sicher und maschinell ablesbar anzeigt.
Die Zellkultur darf neben dem (natürlich vorkommenden oder durch Transfektion eingebrachten) Rezeptor keine oder nur möglichst wenige weitere Kanäle/Rezeptoren mit ähnlicher Funktion oder mit enger Verwandtschaft zu dem zu untersuchenden Kanal/Rezeptor enthalten, um falsch positive Ergebnisse weitgehend auszuschließen.

Als Zellkulturen sind nicht neuronale Zellinien wie die weit verbreitete CHO-Zelle oder HEK-Zelle, aber auch eine Vielzahl von anderen Zellinien wie z.B. Insektenzellinien oder humane Zellinien verwendbar, wenn diese durch Transfektion mit dem Genom für den M-Kanal (KCNQ2/3) oder auch ausschließlich mit der Untereinheit KCNQ2 versehen wurden und diese funktionell exprimieren.
Native Zellinien, die den M-Kanal tragen, wie z.B. die Zellinie PC12 (nach Differenzierung mit dem Wachstumsfaktor NGF) oder die Zellinie NG108-15 (nach Differenzierung zum neuronalen Phänotyp) sind auch verwendbar, tragen aber das Risiko, daß neben dem M-Kanal noch weitere Kaliumkanäle mit ähnlicher Charakteristik exprimiert werden.

Als Reportersysteme sind beispielsweise extern zugegebene kalziumsensitive Fluoreszenzfarbstoffe geeignet. Damit diese Farbstoffe als indirekte Methode zum Nachweis einer Depolarisation angewandt werden können, müssen die Zellsysteme neben den zu untersuchenden Kaliumkanälen auch spannungsabhängig aktivierbare Kalziumkanäle enthalten. Da durch Depolarisation von Zellen (z.B. durch Blockade des M-Kanals) Kalziumkanäle aktiviert werden und dadurch Kalzium in die Zellen einströmt, können bekannte Farbstoffe verwendet werden, die die intrazelluläre Kalziumkonzentration widerspiegeln. Auch können in die zu verwendenden Zellinien durch Transfektion Chemolumineszenzgene eingebracht werden. Die resultierenden Proteine reagieren auf den Einstrom von Kalzium mit Chemolumineszenz. M-Kanal Agonisten lassen sich dadurch detektieren, daß die Membran hyperpolarisiert oder daß eine extem induzierte Depolarisation verhindert wird. Kalziumabhängige Farbstoffe zeigen an, daß aufgrund der fehlenden Depolarisation im Vergleich zur Kontrolle der Kalziumeinstrom ausbleibt.
M-Kanal-Antagonisten lösen selbst eine meßbare Depolarisation aus und führen zum Kalziumeinstrom. Dadurch können in einem solchen System sowohl Agonisten als auch Antagonisten des M-Kanals gefunden werden. Zur Quantifizierung der Wirkung ist es notwendig, daß ein bekannter Standard als Positivkontrolle eingesetzt wird.

Retigabin ist der erste bekannte selektive M-Kanalagonist.

In der vorliegenden Patentanmeldung wird auf folgende Literatur Bezug genommen:
(1) Sewing, S., Röper, J., and Pongs, O.: Structure and function of voltage-gated K⁺ channels. Neuroforum 2, 21-28, 1996.
(2) Schroeder, B.C., Kubisch, C., Stein, V., Jentsch, T.J. Moderate loss of function of cyclic-AMP modulated KCNQ2/KCNQ3 K⁺ channels causes epilepsy. Nature 396, 687-690, 1998.
(3) Wang, H.S., Pan, Z., Shi, W., Brown, B.S., Wymore, R.S., Cohen, U.S., Dixon, J.E., McKinnon, D. KCNQ2 and KCNQ3 potassium channel subunits: molecular correlates of the M-channel. Science 282, 1890-1893, 1998.
(4) Janz, D. (1985) Epilepsy: Seizures and syndromes. In: Frey, H.-H. and Janz, D. eds, Antiepileptic drugs. Handbook of experimental pharmacology, Vol. 74, pp 3-34, Springer-Verlag, Berlin, Heidelberg, NewYork, Tokyo.
(5) Rundfeldt, C., Potassium channels and neurodegenerative diseases. Drug News and Perspectives 12, 99-104, 1999.
(6) Rostock, A., Tober, C., Rundfeldt, C., Bartsch, R., Engel, J., Polymeropoulos, E.E., Kutscher, B., Löscher, W., Hönack, D., White, H.S., and Wolf, H.H.. D-23129: a new anticonvulsant with a broad spectrum activity in animal models of epileptic seizures. Epilepsy. Res. 23, 211-223, 1996
(7) Rundfeldt, C. The new anticonvulsant retigabine (D-23129) acts as an opener of K⁺ channels in neuronal cells, Eur. J. Pharmacol., 336 (1997) 243-249
(8) Rundfeldt, C. Characterization of the K⁺ channel opening effect of the anticonvulsant retigabine in PC12 cells. Epilepsy Res. 35, 99-107, 1999b
(9) Porter, R.J. Classification of epileptic seizures and epileptic syndromes. In: Laidlaw J., Richens A., Chadwick D., (eds): A Textbook of Epilepsy. Churchill Livingstone, New York, 1993, pp. 1-22.

## Patentansprüche

1. Verwendung von Zellkulturen oder Protein-produzierenden Systemen, die natürlicherweise oder durch Transfektion die genetische Information für den Kaliumkanal mit den Untereinheiten KCNQ2 alleine oder in Verbindung mit der Untereinheit KCNQ3 enthalten und diese genetische Information in Protein übersetzen, zur Auffindung von Substanzen, die diesen Kanal über die für Retigabin selektive Bindungsstelle wie Retigabin auch positiv modulieren, d.h. aktivieren.

2. Verwendung von Zellkulturen oder Protein-produzierenden Systemen, die natürlicherweise oder durch Transfektion die genetische Information für den Kaliumkanal mit den Untereinheiten KCNQ2 alleine oder in Verbindung mit der Untereinheit KCNQ3 enthalten und diese genetische Information in Protein übersetzen, zur Auffindung von Substanzen, die diesen Kanal über die für Retigabin selektive Bindungsstelle negativ modulieren, d.h. in seiner Funktion reduzieren.

3. Verwendung von Zellkulturen oder Protein-produzierenden Systemen, die natürlicherweise oder durch Transfektion die genetische Information für den Kaliumkanal mit den Untereinheiten KCNQ2 alleine oder in Verbindung mit der Untereinheit KCNQ3 enthalten und diese genetische Information in Protein übersetzen, zur Auffindung von Substanzen mittels Bindungsassays, die an die für Retigabin selektive Bindungsstelle an diesem Kanal binden.

4. Verwendung von Zellpräparationen zur Herstellung von Fraktionen der Zellpräparation, die nicht mehr die genetische Information für den Kaliumkanal mit den Untereinheiten KCNQ2 alleine oder in Verbindung mit der Untereinheit KCNQ3, wohl aber das der genetischen Information entsprechende Protein enthalten, zur Auffindung von Substanzen, die den Kaliumkanal über die für Retigabin selektive Bindungsstelle positiv oder negativ modulieren.

5. Verwendung von Zellen oder Zellpräparationen in automatisierbaren Screeningsystemen zum Hoch-Durchsatz-Screening unter Verwendung von kalziumsensitiven Fluoreszenzfarbstoffen oder Chemolumineszenzgenen als Reportersystem zur Auffindung von Substanzen, die den Kaliumkanal mit den Untereinheiten KCNQ2 alleine oder in Verbindung mit der Untereinheit KCNQ3 über die für Retigabin selektive Bindungsstelle positiv oder negativ modulieren.

6. Verwendung von Präparationen, die nicht die vollständige genetische Information für den Kaliumkanal mit den Untereinheiten KCNQ2 alleine oder in Verbindung mit der Untereinheit und KCNQ3, wohl aber den Bestandteil der genetischen Information, der die Bindungsstelle für Retigabin kodiert hat, enthalten oder die nur den Teil des Proteins, der die Bindungsstelle für Retigabin enthält, enthalten, zum Aufbau von Systemen zur Auffindung von Substanzen, die den Kaliumkanal mit den Untereinheiten KCNQ2 alleine oder in Verbindung mit der Untereinheit KCNQ3 über die für Retigabin selektive Bindungsstelle gemäß positiv oder negativ modulieren.

7. Verwendung von Zellkulturen oder Protein-produzierenden Systemen, die einen Anteil der genetischen Information des Kanals KCNQ2 alleine oder in Verbindung mit KCNQ3 zusammen mit der genetischen Information für Anteile anderer Kanäle oder Proteine als chimäre genetische Information enthalten, und deren Übersetzung ein chimäres Protein ergibt, das die Bindungsstelle für Retigabin enthält, zum Aufbau von Systemen zur Auffindung von Substanzen, die den Kaliumkanal mit den Untereinheiten KCNQ2 alleine oder in Verbindung mit der Untereinheit KCNQ3 über die für Retigabin selektive Bindungsstelle gemäß positiv oder negativ modulieren.

## Claims

1. Use of cell cultures or protein-producing systems which, naturally or by means of transfection, contain the genetic information for the potassium channel with the subunits KCNQ2 alone or in combination with the subunit KCNQ3 and translate this genetic information into protein, for finding substances which like retigabine also positively modulate i.e. activate this channel via the selective binding site for retigabine.

2. Use of cell cultures or protein-producing systems which, naturally or by means of transfection, contain the genetic information for the potassium channel with the subunits KCNQ2 alone or in combination with the subunit KCNQ3 and translate this genetic information into protein, for finding substances which negatively modulate this channel i.e. reduce its function via the selective binding site for retigabine.

3. Use of cell cultures or protein-producing systems which, naturally or by means of transfection, contain the genetic information for the potassium channel with the subunits KCNQ2 alone or in combination with the subunit KCNQ3 and translate this genetic information into protein, for finding substances by means of binding assays which bind to the selective binding site for retigabine on this channel.

4. Use of cell preparations for preparing fractions of cell preparations which no longer contain the genetic information for the potassium channel with the subunits KCNQ2 alone or in combination with the subunit KCNQ3 but do contain the protein corresponding to the genetic information for finding substances which positively or negatively modulate the potassium channel via the selective binding site for retigabine.

5. Use of cells or cell preparations in automatable screening systems for high throughput screening using calcium-sensitive fluorescent dyes or chemiluminescent genes as reporter systems for finding substances which, via the selective binding site for retigabine, positively or negatively modulate the potassium channel with the subunits KCNQ2 alone or in combination with the subunit KCNQ3.

6. Use of preparations which do not contain the complete genetic information for the potassium channel with the subunits KCNQ2 alone or in combination with the subunit KCNQ3 but do contain the component of the genetic information which has coded for the binding site for retigabine or which only contain the part of the protein which contains the binding site for retigabine for constructing systems for finding substances which, via the selective binding site for retigabine, positively or negatively modulate the potassium channel with the subunits KCNQ2 alone or in combination with the subunit KCNQ3.

7. Use of cell cultures or protein-producing systems which contain a part of the genetic information of the channel KCNQ2 alone or in combination with KCNQ3 together with the genetic information for parts of other channels or proteins as chimeric genetic information, the translation of which results in a chimeric protein which contains the binding site for retigabine, for constructing systems for finding substances for finding substances which, via the selective binding site for retigabine, positively or negatively modulate the potassium channel with the subunits KCNQ2 alone or in combination with the subunit KCNQ3.

## Revendications

1. Utilisation de cultures de cellules ou de systèmes produisant des protéines qui contiennent naturellement ou par transfection; l'information génétique pour le canal de potassium avec les sous-unités KCNQ2 seules ou en relation avec la sous-unité KCNQ3 et qui traduisent cette information génétique en protéine, pour détecter des substances qui modulent positivement, c'est-à-dire activent, ce canal par le biais du site de liaison sélectif de la rétigabine, comme également la rétigabine.

2. Utilisation de cultures de cellules ou de systèmes produisant des protéines qui contiennent naturellement ou par transfection l'information génétique pour le canal de potassium avec les sous-unités KCNQ2 seules ou en relation avec la sous-unité KCNQ3 et qui traduisent cette information génétique en protéine, pour détecter des substances qui modulent négativement ce canal, c'est-à-dire réduisent sa fonction, par le biais du site de liaison sélectif de la rétigabine.

3. Utilisation de cultures de cellules ou de systèmes produisant des protéines qui contiennent naturellement ou par transfection l'information génétique pour le canal de potassium avec les sous-unités KCNQ2 seules ou en relation avec la sous-unité KCNQ3 et qui traduisent cette information génétique en protéine, pour détecter des substances au moyen de tests de liaison qui se lient au site de liaison sélectif de la rétigabine de ce canal.

4. Utilisation de préparations de cellules pour la production de fractions de préparation de cellules qui ne contiennent plus l'information génétique pour le canal de potassium avec les sous-unités KCNQ2 seules ou en liaison avec la sous-unité KCNQ3, mais qui contiennent la protéine correspondante de l'information génétique pour la détection de substances qui modulent positivement ou négativement le canal de potassium par le biais du site de liaison sélectif de la rétigabine.

5. Utilisation de cellules ou de préparations de cellules dans des systèmes de détection automatisables pour une détection à haut rendement en utilisant des colorants fluorescents sensibles au potassium ou des gènes chimioluminescents comme système reporter pour détecter des substances qui modulent positivement ou négativement le canal de potassium avec les sous-unités KCNQ2 seules ou en liaison avec la sous-unité KCNQ3 par le biais du site de liaison sélectif de la rétigabine.

6. Utilisation de préparations qui ne contiennent pas l'information génétique complète pour le canal de potassium avec les sous-unités KCNQ2 ou en liaison avec la sous-unité KCNQ3, mais qui contiennent l'élément constitutif de l'information génétique qui a codé pour le site de liaison de là rétigabine, ou qui contiennent seulement la partie de la protéine contenant le site de liaison de la rétigabine, pour construire des systèmes de détection de substances qui modulent positivement ou négativement le canal de potassium avec les sous-unités KCNQ2 seules ou en liaison avec la sous-unité KCNQ3 par le biais du site de liaison sélectif de la rétigabine.

7. Utilisation de cultures de cellules ou de systèmes produisant des protéines, qui contiennent une proportion d'information génétique du canal KCNQ2 seule ou en relation avec KCNQ3, conjointement avec l'information génétique des proportions relatives à d'autres canaux ou protéines sous forme d'information génétique chimérique, et dont la traduction donne une protéine chimère contenant le site de liaison de la rétigabine pour construire des systèmes de détection de substances qui modulent positivement ou négativement le canal de potassium avec les sous-unités KCNQ2 seules ou en liaison avec la sous-unité KCNQ3 par le biais du site de liaison sélectif de la rétigabine.
